(19) Europäisches Patentamt

European Patent Office

Office européen des brevets

(11) **EP 4 310 101 A1**

(12) **EUROPEAN PATENT APPLICATION**

(43) Date of publication:
**24.01.2024   Bulletin 2024/04**

(21) Application number: **22186476.2**

(22) Date of filing: **22.07.2022**

(51) International Patent Classification (IPC):
**C07K 16/28** *(2006.01)*       **A61P 35/00** *(2006.01)*

(52) Cooperative Patent Classification (CPC):
**C07K 16/2803; A61P 35/00;** C07K 2317/35;
C07K 2317/73; C07K 2317/92

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
• **Emergence Therapeutics AG**
  **47059 Duisburg (DE)**
• **Université d'Aix Marseille**
  **13007 Marseille (FR)**
• **INSTITUT NATIONAL DE LA SANTE ET DE LA RECHERCHE MEDICALE - INSERM**
  **75013 Paris (FR)**
• **Institut Jean Paoli & Irène Calmettes**
  **13009 Marseille (FR)**
• **Centre National de la Recherche Scientifique (CNRS)**
  **75016 Paris (FR)**

(72) Inventors:
• **ELANDS, Jack**
  **1190 Forest (BE)**
• **LHOSPPICE, Florence**
  **29770 Primelin (FR)**
• **PRÉVILLE, Xavier**
  **06330 Roquefort les Pins (FR)**
• **OLIVE, Daniel**
  **13009 Marseille (FR)**
• **LOPEZ, Marc**
  **13009 Marseille (FR)**

(74) Representative: **Weickmann & Weickmann PartmbB**
**Postfach 860 820**
**81635 München (DE)**

Remarks:
The complete document including Reference Table(s) and the Sequence Listing(s) can be downloaded from the EPO website

(54) **NOVEL ANTI-NECTIN-4 ANTIBODIES AND ANTIBODY-DRUG CONJUGATES**

(57) The present invention relates to novel anti Nectin-4 antibodies and antibody-drug conjugates comprising these antibodies, as well as to the use thereof.

EP 4 310 101 A1

**Description**

The present invention relates to novel anti Nectin-4 antibodies and antibody-drug conjugates comprising these antibodies as well as to the use thereof.

BACKGROUND OF THE INVENTION

Nectins are adhesion molecules that help organize epithelial and endothelial junctions and serve as receptors for the entry of the herpes simplex virus, measles virus and poliovirus.

They belong to the immunoglobulin superfamily and are homologs of the poliovirus receptor (PVR / CD155), and for this, have also been called poliovirus receptor bound proteins (PRR). To date, 5 members have been described PVR / CD155, Nectin-1 / PRR1 / CD111, Nectin-2 / PRR2 / CD112, Nectin-3 / PRR3 and Nectin-4 / PRR4. Their ectodomain is made up of three immunoglobulin-like (Ig) -type V, C, C domains that share between 30 and 55% identity in their amino acid sequences.

Expression of Nectin / PRR molecules is generally extensive in tissues, including hematopoietic, neuronal, endothelial and epithelial cells, with the exception of Nectin-3 and -4, which exhibit more restricted expression profiles.

Nectin-4 is a particularly interesting target. It is expressed during fetal development, but its expression decreases and is very restricted in adult tissues in comparison to that of other members of the Nectin family. Nectin-4 is a tumor associated antigen in 83 % of bladder cancers, 78 % of breast cancers (mainly triple negative and ERBB2+), 71 % of pancreatic cancers, 55 % of lung cancers, 57% of ovarian cancers, 59% of head and neck cancers, and 55% of esophageal cancers.

Expression of Nectin-4 in these pathologies is associated with poor prognosis, probably as a consequence of the ability of Nectin-4 to confer to tumor cells *in vitro,* higher capacities of migration, proliferation and formation of metastases. In normal tissues, Nectin-4 is only detected in skin, salivary glands, urinary bladder and esophagus. The recent approval by heath authorities of Enfortumab vedotin for the 2nd line treatment of advanced urothelial cancer has completed the validation of Nectin-4 as a target for the treatment of cancer.

SUMMARY OF THE INVENTION

In the present invention it was found that antibodies capable of binding to a specific epitope on Nectin-4 demonstrate improved anti-cancer activity compared to known anti-Nectin-4 antibodies.

A first aspect of the present invention is a monoclonal antibody or antigen-binding fragment thereof specifically binding to a discontinuous epitope on Nectin-4 consisting of amino acids D57, S58, E60, P133, G135, F137, Q138, and R140 and optionally one or more of G56, G59, E126, R128, P133, and A134 of SEQ ID NO: 104 as determined using Deep Mutational Scanning (DMS),

> provided that the antibody or antibody fragment does not comprise a variable heavy chain (VH) region comprising complementarity-determining regions (CDRs) CDR-H1 according to SEQ ID NO: 7, 21, 35, 49 or 63, CDR-H2 according to SEQ ID NO: 8, 22, 36, 50 or 64, and CDR-H3 according to SEQ ID NO: 9, 23, 37, 51 or 65, and a variable light chain (VL) region comprising complementarity-determining regions (CDRs) CDR-L1 according to SEQ ID NO: 10, 24, 38, 52 or 66, CDR-L2 according to SEQ ID NO: 11, 25, 39, 53 or 67, and CDR-L3 according to SEQ ID NO: 12, 26, 40, 54 or 68,

> in particular wherein the antibody or antibody fragment does not comprise a VH region according to SEQ ID NO: 13 and a VL region according to SEQ ID NO: 14,

> in particular wherein the antibody is not the antibody 15A7.5 disclosed in PCT/EP2022/0586626.

A further aspect of the present invention is an antibody-drug conjugate, comprising

> a monoclonal antibody or antigen-binding fragment thereof as defined herein, and

> a drug conjugated to a reactive amino acid residue on the antibody or antigen-binding fragment, e.g., an amino acid residue having a side chain comprising an amino, hydroxy or thiol group, or a reactive group in the antibody glycan structure.

Further, the invention provides a pharmaceutical composition comprising an antibody or antigen-binding fragment thereof, a vector comprising a nucleic acid encoding the above antibody or antigen-binding fragment thereof, or an antibody-drug-conjugate as defined above.

A further aspect of the present invention is the use of the antibody or antibody-drug conjugate or the pharmaceutical composition as described above in medicine, particularly in human medicine.

A further aspect of the present invention is the use of an antibody-drug conjugate or a pharmaceutical composition as described above in a method for the prevention and/or treatment of a Nectin-4 associated disorder, particularly for the prevention and/or treatment of a Nectin-4 positive cancer and/or Nectin-4 associated inflammation disorder.

A further aspect of the present invention the use of an antibody-drug conjugate or a pharmaceutical composition as described above in a method for the prevention and/or treatment of cancer, particularly for the prevention and/or treatment of a cancer selected from bladder, urothelial, endometrial, cervical, colorectal, liver, thyroid, breast, pancreatic, lung, ovarian head and neck and/or esophagus cancer.

A further aspect of the present invention is a method for the prevention and/or treatment of a Nectin-4 associated disorder, particularly for the prevention and/or treatment of a Nectin-4 positive cancer comprising administering to a subject in need thereof a therapeutically effective amount of an antibody or an antibody-drug conjugate or a pharmaceutical composition as described above.

A further aspect of the present invention is a method for the prevention and/or treatment of cancer, particularly for the prevention and/or treatment of a cancer selected from bladder, urothelial, endometrial, cervical, colorectal, liver, thyroid, breast, pancreatic, lung, ovarian head and neck and/or esophagus cancer comprising administering to a subject in need thereof a therapeutically effective amount of an antibody, or an antibody-drug conjugate or a pharmaceutical composition as described above.

## DETAILED DESCRIPTION OF THE INVENTION

The present inventors have found that anti-Nectin-4 antibodies which bind to a discontinuous epitope on Nectin-4 consisting of amino acids D57, S58, E60, P133, G135, F137, Q138, and R140 and optionally one or more of G56, G59, E126, R128, P133, and A134 of SEQ ID NO: 104 demonstrate improved anti-cancer activity compared to known anti-Nectin-4 antibodies. They specifically bind to Nectin-4 expressed by tumors with a higher affinity in comparison to Nectin-4 expressed by human differentiated keratinocytes. In vitro, this selectivity provides the novel antibodies with lower binding affinity, internalization and cytotoxic activity towards keratinocytes in comparison to tumor cells and in reference to the activity of the HA22 mAb (Enfortumab), in the same assays.

In vivo, this low binding capacity to keratinocytes endows the novel antibodies with a higher half-life due to a lower absorption rate in the skin.

Accordingly, the novel antibodies and, in particular, humanized variants derived therefrom, represent a new way to improve therapeutic index of Nectin-4 positive cancer treatment through lower associated skin toxicity and higher anti-tumor selectivity and efficacy.

Thus, a first aspect of the invention relates to a monoclonal antibody or an antigen-binding fragment thereof specifically binding to a discontinuous epitope on Nectin-4 consisting of amino acids D57, S58, E60, P133, G135, F137, Q138, and R140 and optionally one or more of G56, G59, E126, R128, P133, and A134 of SEQ ID NO: 104 as determined using Deep Mutational Scanning (DMS),

provided that the antibody or antibody fragment does not comprise a variable heavy chain (VH) region comprising complementarity-determining regions (CDRs) CDR-H1 according to SEQ ID NO: 7, 21, 35, 49 or 63, CDR-H2 according to SEQ ID NO: 8, 22, 36, 50 or 64, and CDR-H3 according to SEQ ID NO: 9, 23, 37, 51 or 65, and a variable light chain (VL) region comprising complementarity-determining regions (CDRs) CDR-L1 according to SEQ ID NO: 10, 24, 38, 52 or 66, CDR-L2 according to SEQ ID NO: 11, 25, 39, 53 or 67, and CDR-L3 according to SEQ ID NO: 12, 26, 40, 54 or 68,

in particular wherein the antibody or antibody fragment does not comprise a VH region according to SEQ ID NO: 13 and a VL region according to SEQ ID NO: 14,

in particular wherein the antibody is not the antibody 15A7.5 disclosed in PCT/EP2022/058626.

## DETERMINATION OF THE BINDING EPITOPE

The binding epitope on Nectin-4 is determined using deep mutational scanning (DMS). DMS is a mutagenesis method that aims to perform all possible mono-substitutions on all selected residues within a given protein sequence. The DMS library is obtained in the form of DNA coding for the protein under study. In this library, each DNA strand contains a codon that is mutated with respect to the parental sequence.

In a particular aspect, this DMS DNA library is integrated into an expression plasmid specifically designed to express recombinant proteins on the yeast surface. Yeasts are then transformed and induced to allow the expression of the mono-mutated proteins on their surface. This new library (called display library) is screened by flow cytometry using fluorescent reporters to reveal the expression of the protein (anti-tag fluorescent antibody) as well as the binding of the protein to its partner (fluorescent partner).

For epitope mapping, the ideal case is to have two antibodies with compatible epitopes that can bind together on the same antigen. In this way, each of the two antibodies acts as a conformational control of the mutated antigen for the other antibody. Indeed, mono-substitutions made on the antigen can have 4 types of effects:

1. Loss of affinity for the first antibody, while retaining binding for the second: this is a mutation made within the epitope of the first antibody.
2. Loss of affinity for the second antibody, while retaining binding for the first: this is a mutation in the epitope of the second antibody.
3. Loss of affinity for both antibodies: this is a so-called "destructuring" mutation, which affects the conformation of the antigen and thus prevents the binding of both antibodies.
4. No effect: the mutation is not present in the epitope of one of the two antibodies and does not cause a significant change in the conformation of the antigen.

Following flow cytometry analysis, the yeast population that has lost affinity for the antibody of interest while retaining binding for the second antibody is sorted. The plasmids contained in this yeast population are extracted and sequenced by high-throughput sequencing. Analysis of the sequencing data allows the identification of mutations that have affected the binding of the antibody to its target. Thus, this analysis allows to identify the important positions on the antigen for the binding of the antibody of interest: i.e. its epitope.

An example of an antibody that binds to the above epitope is the monoclonal anti-Nectin-4 antibody 15A7.5 mAb. This antibody and other antibodies described in PCT/EP2022/058626 are explicitly excluded from the scope of protection of the present application.

ANTIBODIES

The antibodies of the invention are monoclonal antibodies (mAb) or monoclonal antibody fragments. If not indicated differently, the term "monoclonal" refers to a single species, i.e., single amino acid composition of antibodies or antibody fragments.

The antibodies provided herein show preferably specific binding to Nectin-4 and no essential or no cross-reactivity to other proteins of the human Nectin-family, in particular Nectin-1, and/or rodent Nectin-4, such as Nectin-4 from rat and/or mouse.

The antigen-binding site of an inventive antibody comprises heavy chain variable domains/regions (VH) and/or antibody light chain variable domains/regions (VL), or pairs of VH/VL.

The term "antigen-binding site" denotes the region(s) of an antibody molecule to which a ligand (e.g., the antigen, i.e., Nectin-4, or antigen fragment of it) actually binds and which is derived from an antibody.

The variable domains/regions denote each of the pair of light and heavy chains, which is involved directly in binding the antibody to the antigen. The variable domain of a heavy chain is abbreviated as "VH" and the variable domain of a light chain is abbreviated as "VL".

An antigen-binding site of an antibody according to the invention can contain six complementarity determining regions (CDRs) which contribute in varying degrees to the affinity of the binding site for the antigen. There are three heavy chain variable domain CDRs (CDR-H1, CDR-H2 and CDR-H3) and three light chain variable domain CDRs (CDR-L1, CDR-L2 and CDR-L3). Also included within the scope of the invention are functional antigen binding sites comprised of fewer CDRs (i.e., where binding specificity is determined by three, four or five CDRs). For example, less than a complete set of 6 CDRs may be sufficient for binding. In some cases, a VH or a VL domain will be sufficient.

According to the present invention, a VH region or the CDRs thereof alone may constitute a complete antigen-binding site. In certain embodiments, the antibody comprises a VH region alone. In other embodiments, the antibody comprises a VH region together with a VL region.

The position of CDRs within a VH or VL region may be defined according to Kabat, et al., Sequences of Proteins of Immunological Interest, 5th Ed. Public Health Service, National Institutes of Health, Bethesda, MD (1991) or the IMGT numbering system, both of which are known to the person skilled in the art. The IMGT numbering has been defined to compare the variable domains whatever the antigen receptor, the chain type, or the species (Lefranc M.-P., "Unique database numbering system for immunogenetic analysis" Immunology Today, 18, 509 (1997); Lefranc M.-P., "The IMGT unique numbering for Immunoglobulins, T cell receptors and Ig-like domains" The Immunologist, 7, 132-136 (1999)). If not stated otherwise, the Kabat system is used herein.

As used herein, the terms "binding" and "specific binding" refer to the binding of the inventive antibody or fragment thereof to an epitope of the Nectin-4 antigen. The measure of the binding strength of an antibody is referred to as affinity. Methods for determining such a binding and/or affinity using in vitro assays are known to the person skilled in the art. According to the present invention, detection with flow cytometry by fluorescence, immuno-histochemistry and/or surface plasmon resonance are described and particularly preferred herein.

The affinity of the binding of an antibody to an antigen is defined by the terms Ka (rate constant for the association of the antibody from the antibody/antigen complex), KD (dissociation constant), and $K_{dis}$ (KD/Ka).

In certain embodiments, the antibody of the invention may be a chimeric antibody, a multispecific antibody, in particular a bispecific antibody, a human antibody, a humanized antibody, or an antigen-binding fragment thereof.

According to the present invention, a "chimeric" antibody refers to an antibody in which a portion of the heavy and/or light chain is derived from a particular source or species, while the remainder of the heavy and/or light chain is derived from a different source or species.

"Multispecific antibodies" bind two or more different epitopes. The epitopes may be on the same or different antigens. A preferred example of a multispecific antibody is a "bispecific antibody" or an antigen-binding fragment thereof which binds two different epitopes. A special subset of bispecific antibodies is a "biparatopic" antibody or antigen-binding fragment, in which each antigen-binding domain recognizes unique, non-overlapping epitopes on the same target antigen. A preferred biparatopic antibody of the present invention binds to at least two different epitopes on Nectin-4, in particular to the epitope defined in claim 1 and to the binding epitope of the antibody HA22 and/or 5A12. For example, a biparatopic antibody of the invention may bind to at least two different epitopes on Nectin-4, in particular to the epitope defined above and to the binding epitope of the antibody HA22 and/or 5A12. The anti-Nectin-4 antibodies HA22 and 5A12 are described, for example, in WO 2018/158398A1, the disclosure of which is incorporated by reference herein.

In preferred embodiments, the antibody of the invention is a humanized antibody.

The term "humanized antibody" or "humanized version of an antibody" refers to antibodies for which both heavy and light chains are humanized as a result of antibody engineering. A humanized chain is typically a chain in which the V-region amino acid sequence has been changed so that, analyzed as a whole, is closer in homology to a human germline sequence than to the germline sequence of the species of origin. For example, a murine CDR may be grafted into the framework region of a human antibody to prepare the "humanized antibody." See, e.g., Riechmann, L., et al., Nature 332 (1988) 323-327; and Neuberger, M. S., et al., Nature 314 (1985) 268-270. Other forms of humanized antibodies encompassed by the present invention are those in which the constant region has been additionally modified or changed from that of the original antibody to generate the properties according to the invention. Humanization assessment is based on the resulting amino acid sequence and not on the methodology per se.

Another preferred embodiment refers to human antibodies.

The term "human antibody", as used herein, is intended to include antibodies having variable and constant regions derived from human germ line immunoglobulin sequences. Human antibodies are well-known in the state of the art (van Dijk, M. A., and van de Winkel, J. G., Curr. Opin. Chem. Biol. 5 (2001) 368-374). Human antibodies can also be produced in transgenic animals (e.g., mice) that are capable, upon immunization, of producing a full repertoire or a selection of human antibodies in the absence of endogenous immunoglobulin production.

The antibody of the present invention may be of any suitable class. The term "class" refers to the type of constant domain or constant region possessed by its heavy chain. As used herein, "constant domain" or "constant region" denotes the sum of the domains of an antibody other than the variable region. The constant region is not directly involved in binding of an antigen but exhibits various effector functions. The antibody may be of any of the five major classes of antibodies, particularly of the five major classes of human antibodies: IgA, IgD, IgE, IgG, and IgM, or any subclass thereof (isotype), e.g., IgG1, IgG2, IgG3, IgG4, IgA1, and IgA2. The heavy chain constant domains that correspond to the different classes of immunoglobulins are called a, $\delta$, $\varepsilon$, $\gamma$ and $\mu$, respectively. According to the present invention, an antibody of the class, particularly of the human class IgG, IgA or IgM or a fragment thereof is particularly suitable.

According to a preferred embodiment, an antibody of the invention is selected from class IgG, particularly from the class of human IgG, e.g., of subclass IgG1, IgG2, IgG3 of IgG4, of class IgM, of class IgA or an antigen-binding fragment thereof.

In certain embodiments, the antibody comprises a constant domain, particularly a heavy chain constant domain, more particularly a heavy chain constant domain of the class IgG, particularly of the class human IgG, e.g., of subclass IgG1, IgG2, IgG3 of IgG4, of class IgM, of class IgA, which has a reduced effector function compared to a wild-type sequence of the same subclass, e.g., which has a reduced binding to the Fc receptor. Examples of heavy chain constant domains with reduced effector functions may contain at least one of the mutations D265C, L234A, L235A, P331S, L234F and L235E of human IgG, e.g., IgG1 or IgG4 sequences.

An "antigen-binding fragment" of an antibody refers to a molecule comprising a portion of an intact antibody that binds the antigen to which the intact antibody binds. Examples of antibody fragments include but are not limited to Fv, Fab, Fab', Fab'-SH, F(ab')$_2$; diabodies; linear antibodies; single-chain antibody molecules (e.g., scFv); and multi-specific antibodies formed from antibody fragments. The term also encompasses a fusion protein, e.g., a fusion protein with a non-immunoglobulin peptide or polypeptide, and a conjugate with a non-proteinaceous structure, e.g., a label or a toxin. The terms "antigen-binding fragment of an antibody (thereof)" and "fragment of an antibody (thereof)" may be used interchangeably herein.

The antibody or the antigen-binding fragment may be mono- or multivalent, i.e., it may comprise a single antigen-binding site or multiple antigen-binding sites. For example, Fab fragments have single antigen-binding site, antibodies of the IgG class or Fv or scFv fragments have two antigen-binding sites and antibodies of the IgM class have 5 antigen-binding sites. The term "antibody" also encompasses hetero-specific antibodies, e.g., hetero-bispecific antibodies, which have different antigen-binding sites, particularly antibodies, which are directed to two different epitopes on the antigen. As used herein, "epitope" is a region of an antigen that is bound by an antibody. The term "epitope" includes any polypeptide determinant capable of specific binding to an antibody.

"Percent (%) amino acid sequence identity" with respect to a peptide or polypeptide sequence is defined as the percentage of amino acid residues in a candidate sequence that are identical with the amino acid residues in the specific peptide or polypeptide sequence, after aligning the sequences and introducing gaps, if necessary, to achieve the maximum percent sequence identity, Alignment for purposes of determining percent amino acid sequence identity can be achieved in various ways that are within the skill in the art, for instance, using publicly available computer software such as BLAST.

Beside a lower binding affinity, the inventive antibodies and fragments provided herein show also preferably lower internalization and/or cytotoxic activity towards keratinocytes in comparison to tumor cells.

According to an especially preferred embodiment, the inventive antibodies and fragments show lower binding affinity, lower internalization and lower cytotoxic activity towards keratinocytes in comparison to tumor cells.

Of course, a specific binding to human Nectin-4 expressed by tumors is preferred. Thus, the antibodies provided herein show preferably specific binding to Nectin-4 and no essential or no cross reactivity to other proteins, in particular to proteins of the human Nectin-family such as Nectin-1.

According to a further aspect, the monoclonal antibody or antigen-binding fragment thereof is characterized by binding to human Nectin-4 expressed by tumors with a higher affinity in comparison to Nectin-4 expressed by human differentiated keratinocytes. For comparison, the binding affinity may be detected by flow cytometry via fluorescence, immuno-histochemistry and/or surface plasmon resonance.

According to a further embodiment, inventive antibodies or antigen-binding fragments thereof comprise a labeling group and/or an effector group being coupled to the antibody or antigen-binding fragment. The labeling group may be, for example, a dye, a paramagnetic, radioactive or fluorogenic group that is detectable upon imaging. A preferred effector group is a therapeutic group, in particular a cytotoxic agent, such as chemotherapeutically active agents, drugs, anti-inflammatory agents, radioactive isotopes, toxins such as topoisomerase poisons, enzymes and fragments thereof such as nucleolytic enzymes, growth inhibitory agents, antibiotics as wells as all suitable anticancer and antitumor agents known to the person skilled in the art. Especially preferred is the topoisomerase poison Camptothecin and derivatives and/or structural analogues thereof like Exatecan as well as derivatives thereof like Deruxtecan.

A preferred embodiment of an antitumor agent relates to antitumor immune stimulating agents including but not restricted to toll-like receptor (TLR) agonists or stimulators of interferon genes (STING) pathways.

According to another preferred embodiment an anti-inflammatory agent may be selected from group comprising steroids and corticosteroids such as glucocorticoids, e.g. cortisol and derivatives thereof, or mineralocorticoids such as aldosterone and derivatives thereof.

Another aspect of the present invention is a combination of at least 2 different monoclonal antibodies or fragments as described herein.

NUCLEIC ACID MOLECULES, VECTORS AND CELLS

Further, the present invention relates to a nucleic acid molecule, e.g. a DNA molecule, encoding an antibody VH region, or an antibody VL region, or encoding a complete antibody or an antibody fragment as indicated above, a vector or vector system, i.e. a plurality of vectors, comprising said nucleic acid molecule(s) as indicated above, preferably in operative linkage with an expression control sequence, particularly with a heterologous expression control sequence.

Furthermore, the invention relates to a cell comprising a nucleic acid molecule or a vector or vector system as described above. The term "vector," as used herein, refers to a nucleic acid molecule capable of propagating another nucleic acid to which it is linked. According to a preferred embodiment, the vector is an expression vector. An "expression vector" is a vector are capable of directing the expression of nucleic acids to which they are operatively linked. Vectors, in particular expression vectors, for the recombinant production of antibodies are well known in the art.

The cell may be a known host cell for producing antibodies or antibody fragments, e.g. a prokaryotic cell such as an E. coli cell, a yeast cell, an insect cell or a mammalian cell, e.g. a CHO cell or a hybridoma cell.

ANTIBODY-DRUG CONJUGATES

An antibody-drug conjugate of the present invention comprises

a monoclonal antibody or antigen-binding fragment thereof as defined above, and
a drug conjugated to a reactive amino acid residue on the antibody or antigen-binding fragment, e.g., an amino acid residue having a side chain comprising an amino, hydroxy or thiol group, or a reactive group in the antibody glycan structure.

The drug of the antibody-drug conjugate of the invention is preferably a topoisomerase I inhibitor. A topoisomerase I inhibitor is a compound, which is capable of forming a ternary complex with topoisomerase I and DNA, thereby preventing DNA re-ligation and introducing DNA strand breaks in the cellular genome. The topoisomerase I inhibitor may be e.g., selected from camptothecin or analogs thereof, indenoisoquinolines and indolocarbazoles.

In a particular embodiment, the topoisomerase-I-inhibitor is camptothecin or an analog thereof, i.e. a compound comprising the pentacyclic basic structure of camptothecin and modified substituents optionally resulting in the presence of a further ring. Specific examples are camptothecin, topotecan, irinotecan, SN-38, belotecan, exatecan including derivatives thereof such as deruxtecan, lurtotecan or atiratecan.

In principle, the drug, e.g. the topoisomerase-I-inhibitor, such as exatecan, may be conjugated to any suitable position of the monoclonal antibody or antigen-binding fragment thereof, particularly to any position, which does not abolish the binding of the antibody to Nectin-4. For example, the drug, e.g. the topoisomerase-I-inhibitor, such as exatecan, may be conjugated to a reactive amino acid residue on the antibody, e.g., an amino acid residue having a side chain comprising an amino, hydroxy or thiol group, or a reactive group in the antibody glycan structure. In particular embodiments, the drug, e.g. the topoisomerase-I-inhibitor, such as exatecan, is conjugated to a reactive thiol group in the side chain of an accessible cysteine residue on the antibody.

In certain embodiments, the antibody drug conjugate comprises has a molar drug-antibody/antibody fragment ratio (DAR) of greater than 1, i.e., more than one drug molecule is attached to an antibody/antibody fragment. Typically, the conjugate has a DAR of about 2:1 to about 16:1, particularly of about 4:1 to about 10:1 and more particularly of about 6:1 to about 8:1. The DAR may be calculated from a statistical distribution according to known methods.

The drug can be conjugated to the antibody or antigen-binding fragment thereof via a linker. In certain embodiments, the linker is a cleavable linker, i.e., a linker cleavable under physiological conditions, e.g., by physiological enzymes. Specific examples of cleavable linkers are peptide-based linkers, which may be subject to cleavage by a protease, or glycoside-based linkers, which may be subject to cleavage by a glycosidase.

In certain embodiments, the linker is a hydrophilic polysarcosine linker e.g., as described by Conilh et at. "Exatecan antibody drug conjugates based on a hydrophilic polysarcosine drug-linker platform" (Pharmaceuticals 14 (2021), 247). Further preferred linkers include linkers comprising at least one ethylene glycol unit, e.g., 1, 2, 3, 4, 5, 6, 7, 8, 9, or 10 or more ethylene glycol units, e.g., the linker of the antibody-drug conjugate MEDI7247, an mcc-triazole spacer-PEG7-x-Lys-PABC glycol linker from Trodelvy®, Further preferred linkers include oligopeptide, particularly di- to decapeptide, e.g., tetrapeptide sequences such as glycine-glycine-phenylalanine-glycine from Enhertu®. Further preferred linkers include highly polar spacers such as an acyl group, carbamoyl group and/or sulfamide group added to at least at least one ethylene glycol unit, e.g., 1, 2, 3, 4, 5, 6, 7, 8, 9, or 10 or more ethylene glycol units, e.g., the linker technology called Hydraspace™. A particularly preferred linker is a β-glucuronide linker.

In certain embodiments, the linker is a hydrophilic polysarcosine linker comprising, e.g., up to 15 sarcosine units, and at least one ethylene glycol unit, e.g., 1, 2, 3, 4, 5, 6, 7, 8, 9, or 10 or more ethylene glycol units, wherein the linker is subject to cleavage by a glycosidase, and particularly subject to cleavage by a glucuronidase.

In certain embodiments, the linker is a hydrophilic polysarcosine linker comprising, e.g., about 8-12 sarcosine units, and at least one ethylene glycol unit, e.g., up to 10 ethylene glycol units, and wherein the linker is subject to cleavage by a glycosidase, and particularly subject to cleavage by a glucuronidase.

In certain embodiments, the linker is a hydrophilic polysarcosine linker comprising 10 sarcosine units, and 2 ethylene glycol units, and wherein the linker is subject to cleavage by a glucuronidase.

The antibody-drug conjugate of the present invention may be prepared by known methods. The antibody or the antigen-binding fragment thereof may be produced in a suitable host cell comprising a nucleic acid molecule, e.g., a DNA molecule, encoding an antibody VH region, or an antibody VL region, or encoding a complete antibody or an antibody fragment, or a vector or vector system, i.e. a plurality of vectors, comprising said nucleic acid molecule(s), preferably in operative linkage with an expression control sequence, particularly with a heterologous expression control sequence. The host cell may be any known host cell for producing antibodies or antibody fragments, e.g., a prokaryotic cell such as an E. coli cell, a yeast cell, an insect cell, or a mammalian cell, e.g., a CHO cell or a hybridoma cell.

The antibody or antigen-binding fragment thereof may be reacted with a linker-drug conjugate to obtain the antibody-drug conjugate. The linker-drug conjugate comprises a drug molecule, e.g., having attached thereto a suitable linker, wherein the linker comprises a reactive group capable of reacting with desired attachment positions on the antibody or antigen-binding fragment thereof. For attachment to cysteine residues, the linker-drug conjugate comprises a thiol-reactive group, e.g., a maleimide group.

## PHARMACEUTICAL COMPOSITIONS

A further aspect of the present invention is a pharmaceutical composition comprising an active agent, which is an antibody or antibody-drug conjugate as herein described and a pharmaceutically acceptable carrier and/or excipient.

Examples of suitable carriers and excipients for formulating antibodies and antibody drug conjugates include saline and aqueous buffer solutions and are well known in the art. Typically, the pharmaceutical composition is adapted for parenteral administration, e.g., for subcutaneous, intramuscular, or intravenous injection or by infusion. In further embodiments, the pharmaceutical composition is adapted for local administration, e.g., for intravesical instillation into the bladder.

Depending on the stage and the severity of the disorder, the pharmaceutical composition may be administered once or several times in a therapeutically effective dose to a subject in need thereof, particularly to a human subject. For example, it may be administered once or several times daily, each second day, two times weekly or weekly for a suitable period, e.g., of at least one week, or at least one month.

## MEDICAL APPLICATIONS

According to a further aspect of the invention, the antibody, antibody-drug conjugate or pharmaceutical composition as described above is used in medicine, including human and veterinary medicine, particularly in human medicine.

In a particular embodiment, the antibody, antibody-drug conjugate or pharmaceutical composition as described above is used in a method for the prevention and/or treatment of a Nectin-4 associated disorder such as cancer and/or an inflammatory disease, particularly for the prevention and/or treatment of a Nectin-4 positive cancer and/or inflammatory disease, more particularly for the prevention and/or treatment of a cancer and/or an inflammatory disease associated with Nectin-4 overexpression.

The cancer to be prevented and/or treated may be any kind of cancer, wherein the term "cancer" is used herein to refer to proliferative diseases. The cancer to be prevented and/or treated according to the present invention is preferably selected from the group consisting of bladder, urothelial, endometrial, cervical, colorectal, liver, thyroid, breast, pancreatic, lung, ovarian head and neck and/or esophagus cancer.

In therapeutic applications, the active agent is administered in an effective amount to a subject in need thereof, particularly to a human subject. The dose will depend on the specific type of agent, e.g., type of antibody or antibody fragment, the type of disease, and the mode of administration, e.g., locally or systemically.

In the prevention and/or treatment of cancer, a therapeutic dose of an antibody or antibody drug conjugate is typically from about 0.3 mg/kg to about 10 mg/kg.

The antibody or antibody-drug conjugate may be administered alone or together with a further active agent, which may be selected from chemotherapeutic agents, e.g., antimetabolites, alkylating agents, intercalating agents, or anti-mitotic agents), inhibitors of specific kinases e.g., tyrosine kinase inhibitors, serine/threonine kinase inhibitors or phosphoinositide kinase inhibitors, immunotherapeutic compounds, e.g., immune checkpoint inhibitors, CAR-T cells, therapeutic vaccines or oncolytic viruses.

Further, the present invention is explained in more detail by the following Tables, Figures and Examples.

## FIGURES

**FIG. 1: Evaluation of the compatibility of antibodies 15A7.5_H1L2 and HA22** Yeasts expressing the Nectin-4 fragment 32-349 were incubated with 100 nM of unlabeled antibody 1 or PBS (as positive control). After 30 minutes incubation, 500 pM of antibody 2 was added for 30 minutes. Yeasts were then incubated with fluorescent reporters to detect by FACS Nectin-4 expression and antibody 2. Results are expressed as percentage of fluorescence to the respective positive control.

**FIG. 2: Libraries sorting by flow cytometry** Two libraries covering the Nectin-4 sequence of interest were generated (left and right panels). Yeasts expressing Nectin-4 mono-mutants were incubated with 100 pM of biotinylated HA22 and 100 pM of 15A7.5_H1L2 labelled with Dy650. Streptavidin PE was used to reveal biotinylated antibodies. After PBS washing, cells were analyzed and sorted on BD FACSAria III as indicated on the graphs. Single-stained yeasts by any antibody, express Nectin-4 mono-mutants that disrupt the epitope for the binding of the other antibody.

**FIG. 3: Positions showing a significant impact on the 15A7.5_H1L2 antibody when mutated.** For any amino acid of the domain of interest, the higher the number of forbidden mutations, the more important the residue for the binding of 15A7.5_H1 L2 antibody.

**FIG. 4: Positions showing a significant impact on the HA22 antibody when mutated.** For any amino acid of the domain of interest, the higher the number of forbidden mutations, the more important the residue for the binding of HA22 antibody.

**FIG. 5: Structure reference of Nectin-4 (4FRW, pdb)** Shown are pymol files. For each antibody, important amino acids of the epitope are indicated. Most important residues are underlined.

**EXAMPLES**

Principle of epitope mapping using DMS (Deep Mutational Scanning)

DMS is a mutagenesis method that aims to perform all possible mono-substitutions on all selected residues within a given protein sequence. The DMS library is obtained in the form of DNA coding for the protein under study. In this library, each DNA strand contains a codon that is mutated with respect to the parental sequence.

This DMS DNA library is integrated into an expression plasmid specifically designed to express recombinant proteins on the yeast surface. Yeasts are then transformed and induced to allow the expression of the mono-mutated proteins on their surface. This new library (called display library) is screened by flow cytometry using fluorescent reporters to reveal the expression of the protein (anti-tag fluorescent antibody) as well as the binding of the protein to its partner (fluorescent partner).

For epitope mapping, the ideal case is to have two antibodies with compatible epitopes that can bind together on the same antigen. In this way, each of the two antibodies acts as a conformational control of the mutated antigen for the other antibody. Indeed, mono-substitutions made on the antigen can have 4 types of effects: (i) loss of affinity for the first antibody, while retaining binding for the second: this is a mutation made within the epitope of the first antibody; (ii) loss of affinity for the second antibody, while retaining binding for the first: this is a mutation in the epitope of the second antibody; (iii) loss of affinity for both antibodies: this is a so-called "destructuring" mutation which affects the conformation of the antigen and thus prevents the binding of both antibodies; (iv) no effect, the mutation is not present in the epitope of one of the two antibodies and does not cause a significant change in the conformation of the antigen. Following flow cytometry analysis, the yeast population that has lost affinity for the antibody of interest while retaining binding for the second antibody is sorted. The plasmids contained in this yeast population are extracted and sequenced by high-throughput sequencing. Analysis of the sequencing data allows the identification of mutations that have affected the binding of the antibody to its target. Thus, this analysis allows to identify the important positions on the antigen for the binding of the antibody of interest: i.e. its epitope.

Construction of the expression plasmid of the antigen

The gene corresponding to the amino acids 32 to 349 of SEQ ID NO: 104 was synthesized and cloned into a plasmid allowing expression on the surface of galactose-inducible yeast. In this construct, the expressed antigen carries a C-terminal HA tag. Expression plasmids are then transformed into the yeast strain S.cerevisiae EBY100. Two DMS libraries were generated by PCR, concerning amino acid positions 32 to 91 of SEQ ID NO: 104 for Library 1 and amino acid positions 92 to 151 of SEQ ID NO: 104 for Library 2. The libraries correspond to IgV domain of Nectin-4, which is known to be the target of the tested antibodies. Each mutated position carries a degenerated NNS or NNK codon encoding 20 amino acids/32 codons. Each library contained approximately 1,200 single amino acid mutants and 2,000 DNA codon mutants. The two libraries were transformed into YSD. Unsorted yeasts from the two generated libraries were sequenced to verify the efficiency of mutagenesis. One hundred percent of the expected single mutants were sequenced for library 1 and 99.9% for library 2.

Epitope compatibility

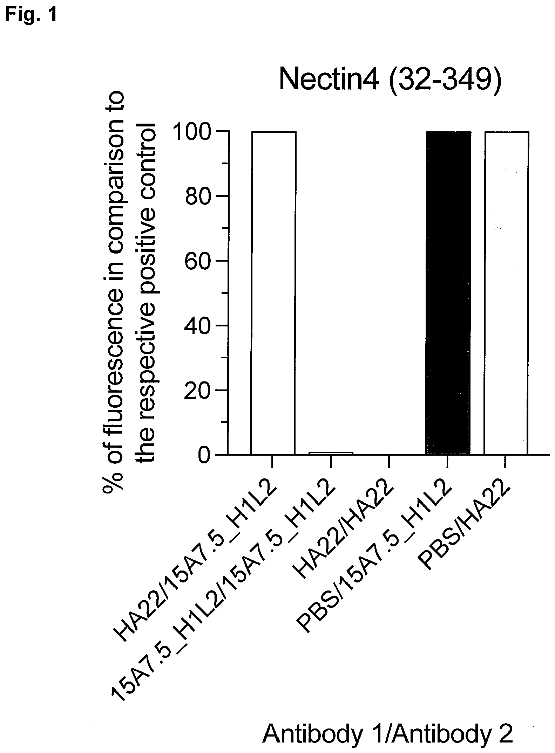

To check if the tested antibodies were compatible for epitope mapping on Nectin-4 IgV domain, i.e. that the antibodies can bind together on Nectin-4 IgV domain, antibodies were first biotinylated (EZ-Link™ Sulfo-NHS-LC-Biotin). Yeasts were induced to express Nectin-4 (32-349) construct and $10^5$ yeasts were washed twice with PBS, BSA 0.1%. Yeast cells were then incubated 30 minutes with 100 nM of unlabeled antibody 1 or left as is for positive control. After 30 minutes, 500 pM of antibody 2 was added for another 30 minutes incubation. Yeasts were then incubated on ice for 15 minutes with respective fluorescent reporters, anti-HA APC for Nectin-4 expression detection and Streptavidin-PE for antibody 2 detection. After washing in PBS-BSA, resuspended yeasts were analyzed by flow cytometry. Figure 1 shows that 15A7.5_H1L2 mAb can bind Nectin-4 IgV domain simultaneously with HA22.

Induction of antigen expression and FACS-sorting of libraries

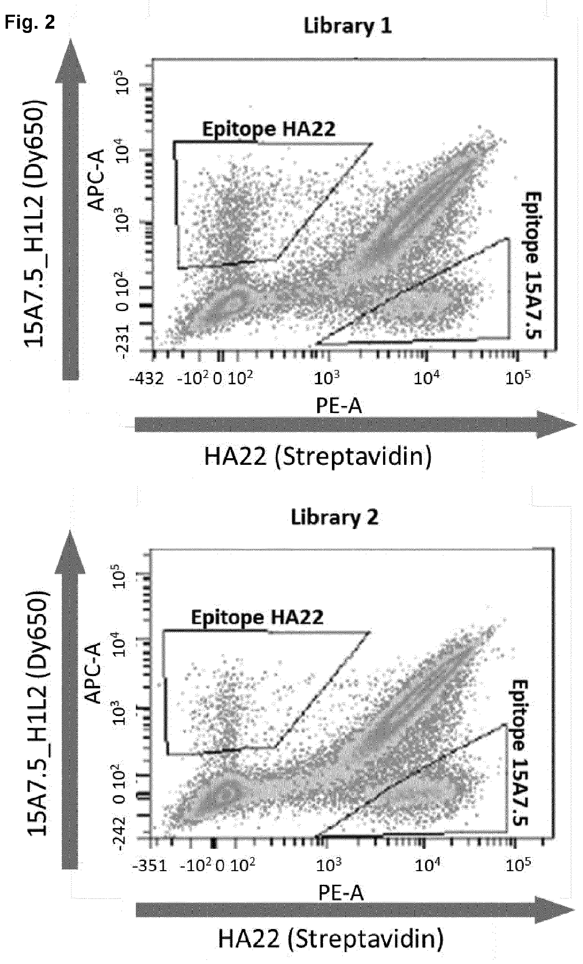

Yeasts expressing Nectin4 mono-mutants were induced for protein expression. Induction of transformed yeast was done in SG-CAA induction medium [6.7 g/L yeast nitrogen base without casamino acids, 20 g/L glucose, 5 g/L casamino acids, 100 mM sodium phosphate, pH 6.0]. For cytometric analysis/sorting, between $10^6$ and $10^8$ induced cells were washed with 1 mL of PBSF (PBS, 0.1% BSA). The cells were then resuspended in an appropriate volume of solution containing 100 pM of the biotinylated HA22 antibody and Dye650-labeled 15A7.5_H1L2. After 1h to 3h of incubation at 20°C with agitation, the cells were washed with 1 mL of ice-cold PBSF and then incubated with the streptavidin on ice for 15 min. The cells are then analyzed/sorted on a BD FACSAria™ III cytometer using the BD FACSdiva™ Diva software. As shown in Figure 2, the single-stained yeasts in each experiment were sorted and plasmids from each sorted yeast population were extracted and prepared for sequencing. A two-step PCR was performed: a first step to amplify the region of interest and a second step to add the Illumina adapters needed for sequencing. Sequencing was performed on an Illumina iSeq100 instrument (2x150 bp, 300 cycles) with at least 150,000 reads per population. The data were then processed through an analysis pipeline using dedicated proprietary scripts. Poor quality sequences (Q<30) were removed, then mono-mutants were detected and counted. For each mono-mutation, the frequency measured in the unsorted population was compared to the frequency of the mutant in the sorted population. An enrichment value for the mutant was then calculated according to the following formula:

$$Enrichment\ mutant = \frac{Frequency\ mutant,\ sorted\ population}{Frequency\ mutant, unsorted\ population}$$

a high enrichment value indicates that the mutation under consideration caused a loss of recognition of the antibody for its antigen, while maintaining recognition of the other antibody (conformational control).

Data interpretation

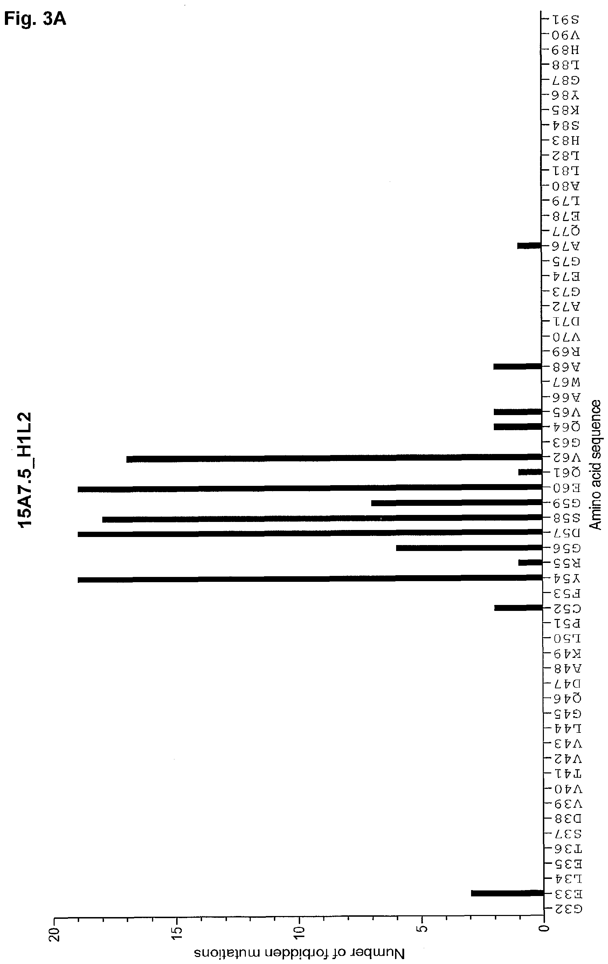

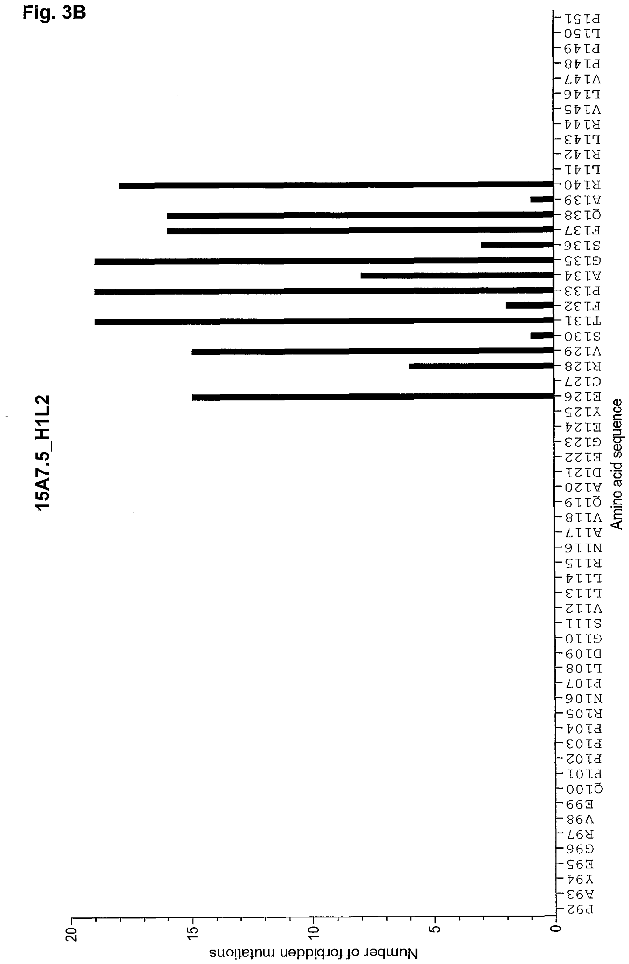

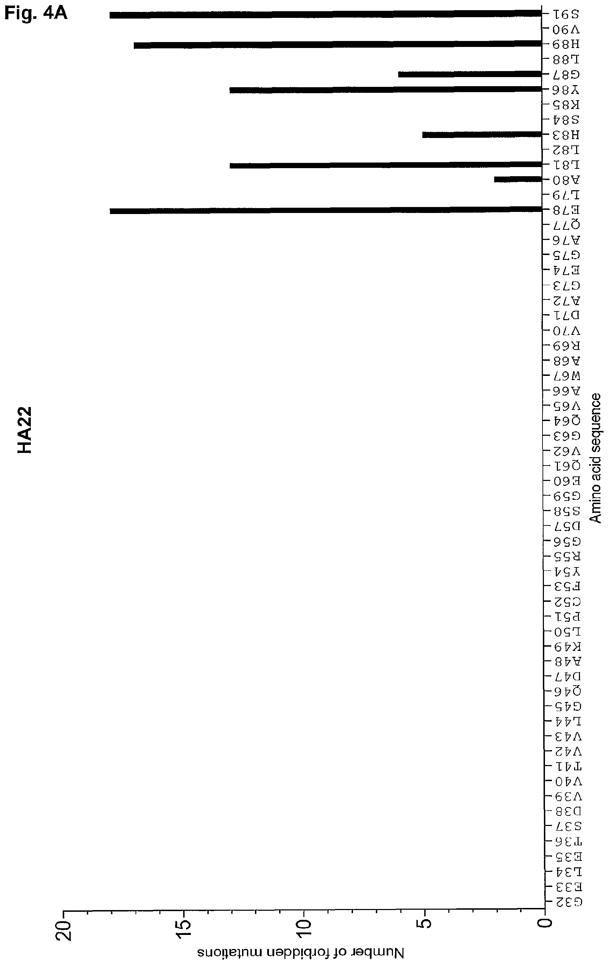

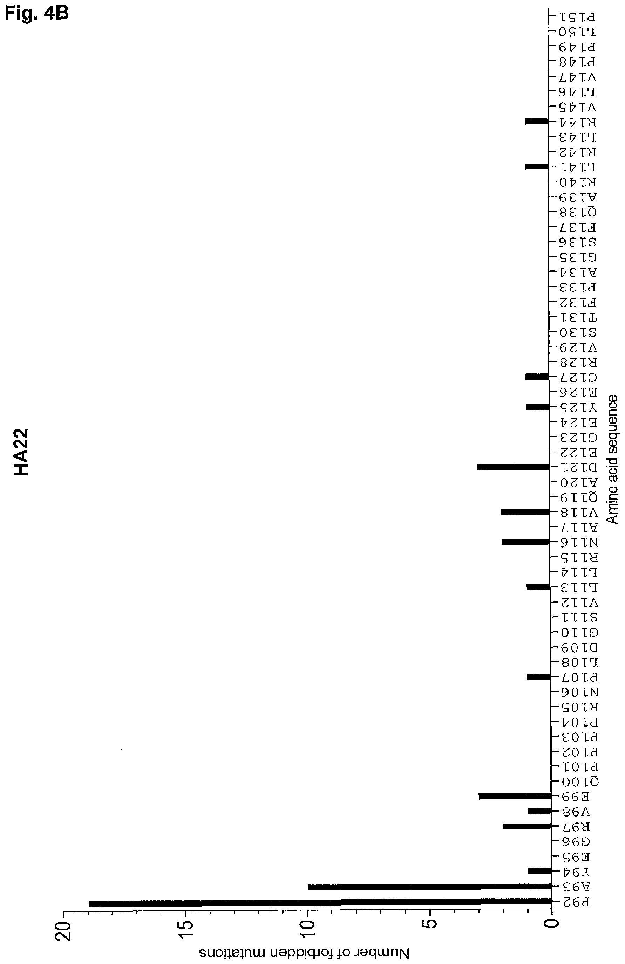

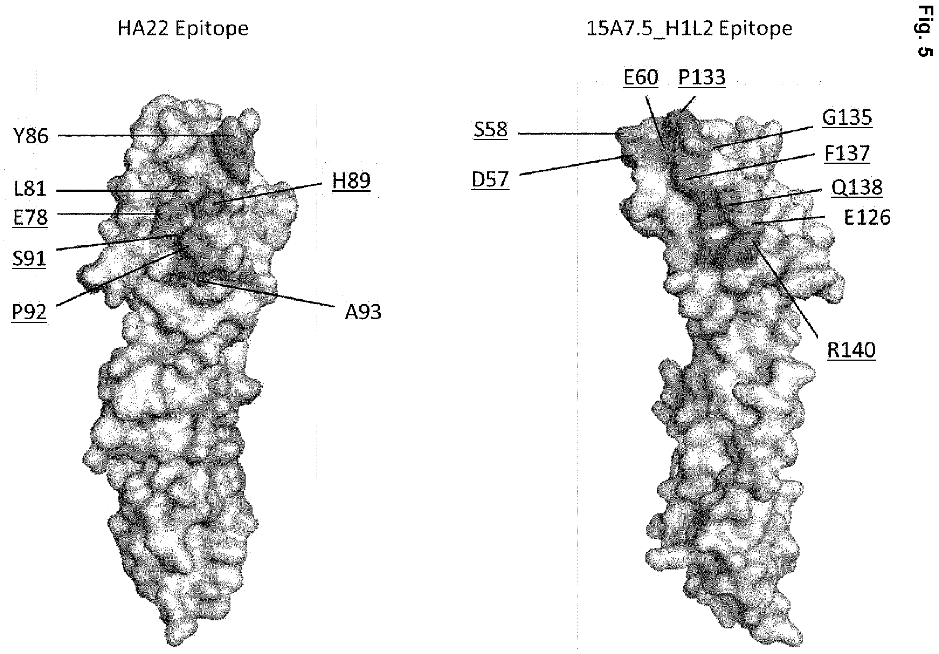

The result of the processing of the NGS sequencing data is represented as a bar graph that aggregates the enrichment values for all single mutants present in the DMS libraries (Figures 3 and 4). Between 16 and 19 forbidden mutations indicate high impact and positions classified in this category are the most likely to be indirect interaction with the considered IgG. Between 10 and 15 forbidden mutations indicate medium impact positions, and between 5 and 9 forbidden mutations indicate low impact positions. Figure 5 shows an analysis of the structure of Nectin-4 (4FRW, pdb) to highlight the important and most important epitope residues for each of the antibody tested. Table 1 lists these residues.

Table 1:

| Antibody | Epitope residues | Most important residues |
|---|---|---|
| 15A7.5_H1L2 | G56, D57, S58, G59, E60, E126, R128, P133, A134, G135, F137, Q138 and R140 | D57, S58, E60, P133, G135, F137, Q138 and R140 |
| HA22 | E78, L81, H83, Y86, G87, H89, S91, P92 and A93 | E78, H89, S91 and P92 |

**Claims**

1. A monoclonal antibody or antigen-binding fragment thereof specifically binding to a discontinuous epitope on Nectin-4 consisting of amino acids D57, S58, E60, P133, G135, F137, Q138, and R140 and optionally one or more of G56, G59, E126, R128, P133, and A134 of SEQ ID NO: 104 as determined using Deep Mutational Scanning (DMS),

   provided that the antibody or antibody fragment does not comprise a variable heavy chain (VH) region comprising complementarity-determining regions (CDRs) CDR-H1 according to SEQ ID NO: 7, 21, 35, 49 or 63, CDR-H2 according to SEQ ID NO: 8, 22, 36, 50 or 64, and CDR-H3 according to SEQ ID NO: 9, 23, 37, 51 or 65, and a variable light chain (VL) region comprising complementarity-determining regions (CDRs) CDR-L1 according to SEQ ID NO: 10, 24, 38, 52 or 66, CDR-L2 according to SEQ ID NO: 11, 25, 39, 53 or 67, and CDR-L3 according to SEQ ID NO: 12, 26, 40, 54 or 68,

in particular wherein the antibody or antibody fragment does not comprise a VH region according to SEQ ID NO: 13 and a VL region according to SEQ ID NO: 14,
in particular wherein the antibody is not the antibody 15A7.5 disclosed in PCT/EP2022/0586626.

2. The antibody or antigen-binding fragment of claim 1, which is a chimeric antibody, a multispecific antibody, in particular a bispecific antibody, a human antibody, a humanized antibody or an antigen-binding fragment thereof.

3. The antibody or antigen-binding fragment of claim 1 or 2, which is a biparatopic antibody or antigen-binding fragment, binding to at least two different epitopes on Nectin-4, in particular to the epitope defined in claim 1 and to the binding epitope of the antibody HA22 and/or 5A12.

4. The antibody or antigen-binding fragment of any one of claims 1 to 3, which is an antibody of class IgG, e.g. of subclass IgG1, IgG2, IgG3 of IgG4, of class IgM, of class IgA or an antigen-binding fragment thereof, or which is a single-chain antibody, or an antibody Fv fragment.

5. The antibody or antigen-binding fragment of any one of claims 1 to 4, binding to Nectin-4 expressed by tumors with a higher affinity in comparison to Nectin-4 expressed by human differentiated keratinocytes.

6. The antibody or antigen-binding fragment of claim 5, wherein the binding affinity is determined by flow cytometry via fluorescence, immuno-histochemistry and/or surface plasmon resonance.

7. The antibody or antigen-binding fragment of any one of claims 1 to 6, comprising a labeling group and/or an effector group coupled to the antibody or antigen-binding fragment.

8. The antibody or antigen-binding fragment of claim 7, wherein the labeling group is a dye, a paramagnetic, radioactive or fluorogenic group that is detectable upon imaging.

9. The antibody or antigen-binding fragment of claim 7, wherein the effector group is a therapeutic group, in particular a cytotoxic agent.

10. The antibody or antigen-binding fragment of any one of claims 1-9 for use in medicine, particularly for therapeutic or diagnostic applications including in vitro and in vivo diagnostic applications.

11. The antibody or antigen-binding fragment for use according to claim 10 in a method of preventing and/or treating cancer and/or inflammatory disorders.

12. The antibody or antigen-binding fragment for use according to claim 11, wherein the cancer is associated with Nectin-4 overexpression.

13. The antibody or antigen-binding fragment for use according to any one of claims 11-12, wherein the cancer is urothelial, endometrial, cervical, colorectal, liver, thyroid, breast, pancreatic, lung, ovarian head and neck and/or esophagus cancer.

14. A nucleic acid encoding the antibody or antigen-binding fragment of anyone claims 1-9.

15. A vector, in particular an expression vector, comprising the nucleic acid of claim 14.

16. A host cell comprising the vector of claim 15.

17. An antibody-drug conjugate, comprising

a monoclonal antibody or antigen-binding fragment thereof according to any one of claims 1-9, and
a drug conjugated to a reactive amino acid residue on the antibody or antigen-binding fragment, e.g., an amino acid residue having a side chain comprising an amino, hydroxy or thiol group, or a reactive group in the antibody glycan structure.

18. The antibody-drug conjugate of claim 17, wherein the drug is conjugated to a reactive thiol group in the side chain of a cysteine residue on the antibody or antigen-binding fragment thereof.

**19.** The antibody-drug conjugate of claim 17 or 18, wherein the drug is conjugated to the antibody or antigen-binding fragment thereof via a linker, particularly wherein the linker is a cleavable linker, such as a linker subject to cleavage by a glucuronidase, and/or wherein the linker is a peptidic linker.

**20.** A pharmaceutical composition comprising an antibody or antigen-binding fragment thereof according to any one of claims 1-9, a vector according to claim 15, or an antibody-drug-conjugate according to any one of claims 17-19.

**Fig. 1**

**Fig. 2**

**Fig. 3A**

**Fig. 3B**

15A7.5_H1L2

Fig. 4A

HA22

**Fig. 4B**

HA22

**Fig. 5**

Europäisches
Patentamt

European
Patent Office

Office européen
des brevets

# EUROPEAN SEARCH REPORT

**Application Number**

EP 22 18 6476

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2021/069508 A1 (UNIV AIX MARSEILLE [FR]; INST NAT SANTE RECH MED [FR] ET AL.) 15 April 2021 (2021-04-15) * Antibody 5A12, N41, 14A5.2; page 29 – page 31; figures 1-3 * | 1,2,4-20 | INV. C07K16/28 A61P35/00 |
| X,D | WO 2018/158398 A1 (INST NAT SANTE RECH MED [FR] ET AL.) 7 September 2018 (2018-09-07) * antibody 14A5.2; figure 2; sequences 1,2 * | 1,2,4-20 | |
| X | WO 2017/042210 A1 (INSERM [FR]; UNIVERSITÉ D'AIX MARSEILLE [FR] ET AL.) 16 March 2017 (2017-03-16) * antibody N41; page 44, line 9 – line 14; sequences 1-2 * | 1,2,4-20 | |
| X | CN 114 702 588 A (BOJI BIOMEDICAL TECH HANGZHOU CO LTD) 5 July 2022 (2022-07-05) * claims 1-4,6-7; sequences 15-20 * | 1,2,4-20 | |
| X | WO 2022/057651 A1 (SICHUAN KELUN BIOTECH BIOPHARMACEUTICAL CO LTD [CN]) 24 March 2022 (2022-03-24) * claims 9,14,27 * | 1,2,4-20 | TECHNICAL FIELDS SEARCHED (IPC) C07K A61P A61K |
| X | WO 2021/168274 A1 (SILVERBACK THERAPEUTICS INC [US]) 26 August 2021 (2021-08-26) * claims 1-10; figures 1-3 * | 1,2,4-20 | |
| X | WO 2018/226578 A1 (AGENSYS INC [US]) 13 December 2018 (2018-12-13) * page 175 – page 182 * | 1,2,4-20 | |

-/--

The present search report has been drawn up for all claims

| Place of search | Date of completion of the search | Examiner |
|---|---|---|
| Munich | 19 December 2022 | Renggli-Zulliger, N |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches Patentamt

European Patent Office

Office européen des brevets

## EUROPEAN SEARCH REPORT

**Application Number**

**EP 22 18 6476**

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | P. M. CHALLITA-EID ET AL: "Enfortumab Vedotin Antibody-Drug Conjugate Targeting Nectin-4 Is a Highly Potent Therapeutic Agent in Multiple Preclinical Cancer Models", CANCER RESEARCH, vol. 76, no. 10, 15 May 2016 (2016-05-15), pages 3003-3013, XP055385700, US ISSN: 0008-5472, DOI: 10.1158/0008-5472.CAN-15-1313 * the whole document * | 1-20 | |
| A | WU SAM ET AL: "Cutaneous toxicity associated with enfortumab vedotin treatment of metastatic urothelial carcinoma", DERMATOLOGY ONLINE JOURNAL, UNIVERSITY OF CALIFORNIA * ESCHOLARSHIP, US, vol. 25, no. 2, 15 February 2019 (2019-02-15), XP002804424, ISSN: 1087-2108 * the whole document * | 1-20 | |

TECHNICAL FIELDS SEARCHED (IPC)

The present search report has been drawn up for all claims

| Place of search | Date of completion of the search | Examiner |
|---|---|---|
| Munich | 19 December 2022 | Renggli-Zulliger, N |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 22 18 6476

This annex lists the patent family members relating to the patent documents cited in the above-mentioned European search report.
The members are as contained in the European Patent Office EDP file on
The European Patent Office is in no way liable for these particulars which are merely given for the purpose of information.

19-12-2022

| Patent document cited in search report | | Publication date | Patent family member(s) | | Publication date |
|---|---|---|---|---|---|
| WO 2021069508 | A1 | 15-04-2021 | AU | 2020364959 A1 | 07-04-2022 |
| | | | CA | 3156451 A1 | 15-04-2021 |
| | | | CN | 114514246 A | 17-05-2022 |
| | | | EP | 4041765 A1 | 17-08-2022 |
| | | | IL | 291966 A | 01-06-2022 |
| | | | JP | 2022551537 A | 09-12-2022 |
| | | | KR | 20220079614 A | 13-06-2022 |
| | | | WO | 2021069508 A1 | 15-04-2021 |
| WO 2018158398 | A1 | 07-09-2018 | CN | 110392697 A | 29-10-2019 |
| | | | EP | 3589654 A1 | 08-01-2020 |
| | | | JP | 2020510432 A | 09-04-2020 |
| | | | US | 2021324104 A1 | 21-10-2021 |
| | | | WO | 2018158398 A1 | 07-09-2018 |
| WO 2017042210 | A1 | 16-03-2017 | EP | 3347048 A1 | 18-07-2018 |
| | | | ES | 2794557 T3 | 18-11-2020 |
| | | | JP | 6985252 B2 | 22-12-2021 |
| | | | JP | 2018531913 A | 01-11-2018 |
| | | | US | 2018243434 A1 | 30-08-2018 |
| | | | WO | 2017042210 A1 | 16-03-2017 |
| CN 114702588 | A | 05-07-2022 | NONE | | |
| WO 2022057651 | A1 | 24-03-2022 | NONE | | |
| WO 2021168274 | A1 | 26-08-2021 | EP | 4106819 A1 | 28-12-2022 |
| | | | TW | 202144010 A | 01-12-2021 |
| | | | US | 2021275683 A1 | 09-09-2021 |
| | | | US | 2022105196 A1 | 07-04-2022 |
| | | | WO | 2021168274 A1 | 26-08-2021 |
| WO 2018226578 | A1 | 13-12-2018 | BR | 112019025513 A2 | 23-06-2020 |
| | | | CA | 3065514 A1 | 13-12-2018 |
| | | | CN | 111051345 A | 21-04-2020 |
| | | | CN | 111675761 A | 18-09-2020 |
| | | | EP | 3635013 A1 | 15-04-2020 |
| | | | JP | 7168590 B2 | 09-11-2022 |
| | | | JP | 2020522261 A | 30-07-2020 |
| | | | KR | 20200024788 A | 09-03-2020 |
| | | | RU | 2019144030 A | 09-07-2021 |
| | | | TW | 201902922 A | 16-01-2019 |
| | | | US | 2020231670 A1 | 23-07-2020 |
| | | | WO | 2018226578 A1 | 13-12-2018 |

EPO FORM P0459

For more details about this annex : see Official Journal of the European Patent Office, No. 12/82

## REFERENCES CITED IN THE DESCRIPTION

**Patent documents cited in the description**

- EP 20220586626 W
- EP 2022058626 W

- WO 2018158398 A1


**Non-patent literature cited in the description**

- **KABAT et al.** Sequences of Proteins of Immunological Interest. Public Health Service, National Institutes of Health, 1991
- **LEFRANC M.-P.** Unique database numbering system for immunogenetic analysis. *Immunology Today,* 1997, vol. 18, 509
- **LEFRANC M.-P.** The IMGT unique numbering for Immunoglobulins, T cell receptors and Ig-like domains. *The Immunologist,* 1999, vol. 7, 132-136

- **RIECHMANN, L. et al.** *Nature,* 1988, vol. 332, 323-327
- **NEUBERGER, M. S. et al.** *Nature,* 1985, vol. 314, 268-270
- **VAN DIJK, M. A. ; VAN DE WINKEL, J. G.** *Curr. Opin. Chem. Biol.,* 2001, vol. 5, 368-374